(19) Europäisches Patentamt
European Patent Office
Office européen des brevets

(11) Publication number: 0 273 893
A2

# (12) EUROPEAN PATENT APPLICATION

(21) Application number: **87850397.8**

(22) Date of filing: **23.12.87**

(51) Int. Cl.⁴: **C 07 K 5/06**
C 07 C 147/05,
C 07 C 149/24,
C 07 D 233/64, A 61 K 37/64,
A 61 K 31/165, C 07 D 263/24

(30) Priority: **29.12.86 SE 8605573**

(43) Date of publication of application:
**06.07.88 Bulletin 88/27**

(84) Designated Contracting States:
**AT BE CH DE ES FR GB GR IT LI LU NL SE**

(71) Applicant: **Aktiebolaget Hässle**
**Kärragatan 5**
**S-431 83 Mölndal (SE)**

(72) Inventor: **Karlsson, Jan Olle**
**Gundefjällsgatan 377**
**S-431 45 Mölndal (SE)**

**Sohtell, Erik Morgan Herman**
**Lindomebyvägen 27**
**S-437 00 Lindome (SE)**

**Starke, Carl Ingemar**
**Kristinehöjdsgatan 15**
**S-412 82 Göteborg (SE)**

**Westerlund, Rolf Christer**
**Pepparreds torg 12**
**S-431 45 Mölndal (SE)**

(74) Representative: **Näsman, Rune B. G. et al**
**AB Astra Patent and Trade Mark Department**
**S-151 85 Södertälje (SE)**

(54) **Novel compounds.**

(57) Compounds having a renin inhibitory effect and having the formula

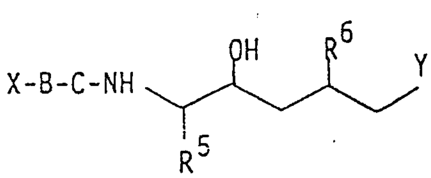

are disclosed wherein X is H or a N-protecting group
$R^1-(CH_2)_n-O-C(O)-$
where n is O-4; $R^1$ is a straight or branched alkyl group containing 1-6 carbon atoms
and
B is phenylalanyl; O-methyltyrosyl; homophenylalanyl; cyclohexylalanyl; dibenzylacetyl
and
C is norvalyl; valyl; norleucyl; leucyl; histidyl; either as such or N-alkylated
and
$R^5$ is a straight or branched alkyl group containing 1-6 carbon atoms or a cyclohexylmethyl group
and $R^6$ is H or a stright or branched alkyl group containing 1-6 carbon atoms
and
Y is selected from
a) $-SCH(CH_3)_2$
b) $-S(O)_2CH(CH_3)_2$
either as such or in the form of physiologically acceptable salts and including optical isomers. Processes for preparation and pharmaceutical preparations for such compounds and methods for treatment of heart disorders with such compounds are further disclosed.

EP 0 273 893 A2

**Description**

## Novel compounds

Background

Renin is a natural proteolytic enzyme, which is released into the blood from the kidney. Its only known function is cleave circulating angiotensinogen into angiotensin I, which is a decapeptide of the following structure:

Asp-Arg-Val-Tyr-Ile-His-Pro-Phe-His-Leu

This in turn is further cleaved by angiotensin converting enzyme (ACE), resulting in the formation of the octapeptide angiotensin II:

Asp-Arg-Val-Tyr-Ile-His-Pro-Phe

This peptide is one of the most potent blood-pressure elevating agents known. Its biological activity in this respect is partly due to a vasoregulating effect and partly on an aldosterone mediated antidiuretic and antisaliuretic effect.

Efforts to control hypertension by way of the reninangiotensin system has until lately been mainly directed towards inhibition of ACE. Although this principle has proved clinically useful, the non-specificity of ACE has been advocated as a reason for different side-effects. Since renin is highly specific for angiotensinogen, inhibitors of this enzyme could prove advantageous in the combat against different forms of hypertension.

Prior art

The earliest efforts towards making renin inhibitors was mainly directed towards simple substrate analogues. By variations in the amino acid sequence Haber et al succeeded in increasing their potency. Further effectiveness was achieved by replacing the scissile dipeptide unit in the inhibitors with a non-cleavable unit such as statine (Boger et al Nature, 3O3, 81 (1983) or different isosteres (Szelke et al Nature, 299, 555 (1982). Compounds of this type proved very potent but had short duration of effect. This was attributed to proteolytic instability of the peptides, and thus much of the more recent work in this area has been directed towards making smaller inhibitors with reduced number of potentially scissile peptide bonds. For some recent publications on this subject see Matsuda et al, Chem. Lett. 1O41 (1985), Plattner et al, 191th ACS-meeting, New York, (1986), Hanson et al, Biochem. Biophys. Res. Comm. 132, 155 (1985) and Toda et al, Eur. J. Pharm. 129, 393 (1986). Recent patent applications relating to shorter inhibitors include EP 186977 (Sankyo), EP 189182 (Abbott), EP 19O891 (Kissei), EP 172346 (Abbott), EP 1892O3 (Abbott), EP 172347 (Abbott) and EP 18111O (Kissei).

Definitions and abbreviations

Definitions

The following definitions apply both to the descriptions of the invention and the claims unless otherwise specified.

1. The term "amino acid" includes amino acids of both natural and unnatural origin.
2. All amino acids are can be of either L- or D-configuration but are preferably of the L-configuration unless stated.
3. All asymmetric centers may be of either R or S configuration unless otherwise stated.

Abbrevations

Asp = Aspartic acid
Arg = Arginine
Val = Valine
Tyr = Tyrosine
Ile = Isoleucine
His = Histidine
Pro = Proline
Phe = Phenylalanine
Leu = Leucine
Nva = Norvaline
Nle = Norleucine
Cha = Cyclohexylalanine
Ala = Alanine
Trp = Tryptophan
Abu = 2-Aminobutyric acid
Tyr(Me) = O-methyltyrosine
hPhe = Homophenylalanine
Me-Nva = N-methylnorvaline
Me-Nle = N-methylnorleucine

Boc = t-Butoxycarbonyl
Dba = Dibenzylacetyl
Z = Benzyloxycarbonyl
Su = Succinimido

Disclosure of the invention

This invention relates to new types of short renin inhibitors, their synthesis, pharmaceutical composition containing the compounds as active ingredients and the use of the compounds as drugs for treatment of hypertension, congestive heart failure and other cardiovascular disorders.

Thus it has been found that compounds of the general formula

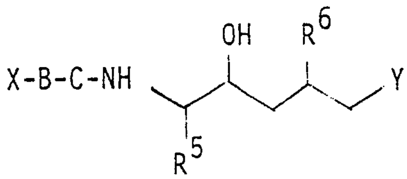

where
X = H or an N-protecting group
$R^1-(CH_2)_n-O-C(O)-$
where n=O-4; $R^1$ = a straight or branched alkyl group containing 1-6 carbon atoms
and
B = phenylalaninyl; O-methyltyrosinyl; homophenylalaninyl; cyclohexylalaninyl; dibenzylacetyl
and
C = norvalinyl; valinyl; norleucinyl; leucinyl; histidinyl; either as such or N -alkylated
and
$R^5$= a straight or branched alkyl group containing 1-6 carbon atoms; cyclohexylmethyl
and
$R^6$ =H; straight or branched alkyl containing 1-6 carbon atoms
and
Y =

a) $-SCH(CH_3)_2$
b) $-S(O)_2CH(CH_3)_2$

either as such or in the form of physiologically acceptable salts and including optical isomers, show high and specific renin inhibitory effects.

Preferred compounds are those where X is selected from Boc, H;B is selected from Phe, Tyr(Me), Cha, hPhe and 6a; C is selected from His, Nle, Nva, Me-Nva, Me-Nle and Me-His; $R^5$ is a cyclohexylmethyl group; and $R^6$ is methyl. Specific preferred compounds are those shown in Table 1.

A further objective of the invention is the mode of preparation of the compounds of the invention. Thus the syntheses of the compounds

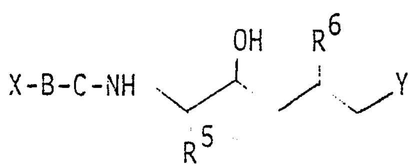

are essentially achieved by first assembling the

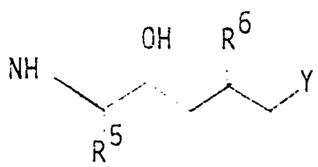

portion henceforth referred to as the isostere, followed by one or two coupling reactions, optionally followed or intervened by some further manipulations of the isostere part, thus leading to structures of the said type.

I Synthesis of isosteres.

The isosteres are prepared according the following methods

Step 1

Method A

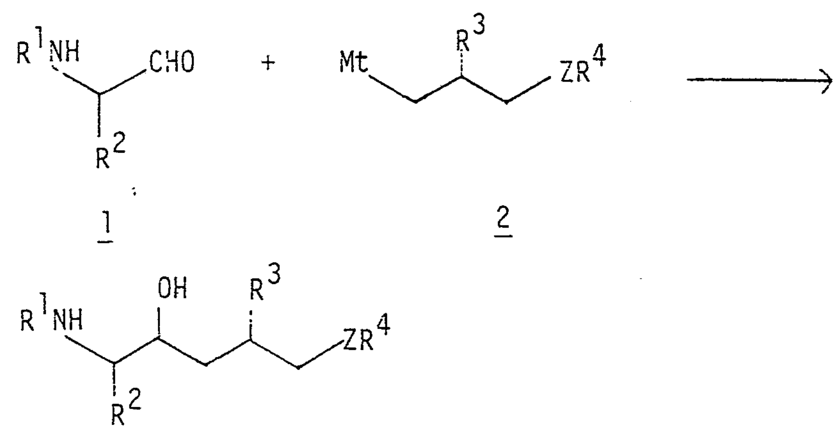

1

2

3

where an organometallic reagent 2 attacks an amino aldehyde 1 in a protected form to give compound 3 (Z=O).

Method B

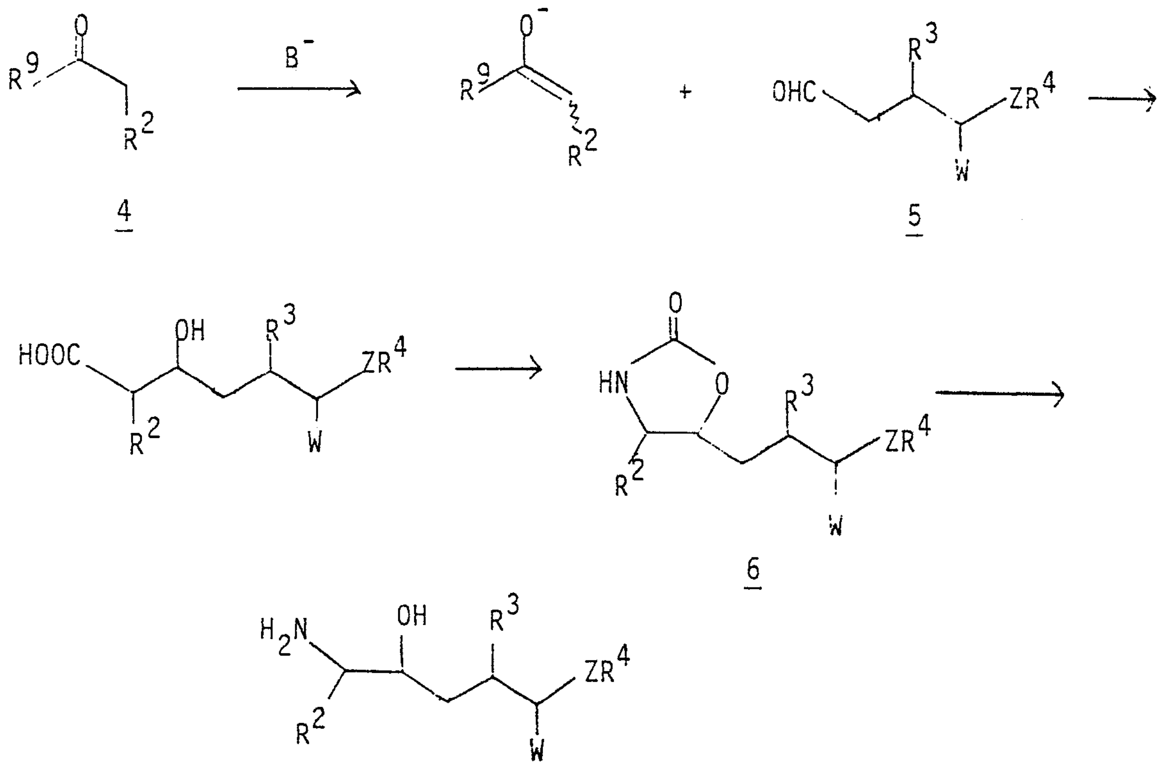

4 5 6 3a

where an appropriate carboxylic acid derivative 4 and an aliphatic aldehyde 5 react in an aldol reaction producing, after a Curtius rearrangement of the liberated carboxylic acid, an oxazolidinone 6 which can be cleaved by alkaline hydrolisis to give the desired amino alcohol 3a (Z=O).

Step 2

Conversion of Z=O into Z= S(O)n

The oxygen is introduced as an ether or ester. At some step in the synthesis cleavage of the ethers and reduction of the esters respectively yields the alcohol 22.

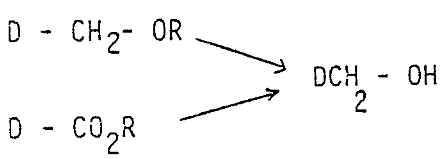

22

The oxygen in 22 is replaced by sulfur by first transforming the primary alcohol into a leaving group.

The sulfur is then introduced by treating 24 with a thiolate. The resulting thioether 28 can be oxidized to sulfone 3O prior to or after the coupling of the amino acids.

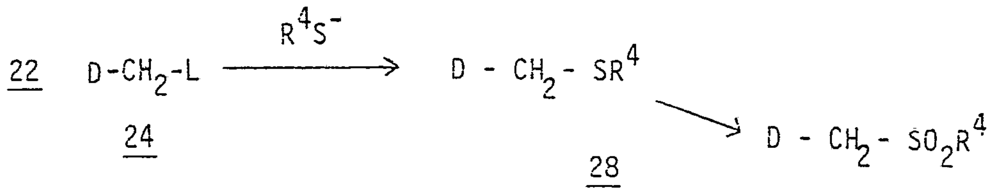


22 24 28 30

Certain precautions have to be taken when 24 is synthesized:
In these hydroxy isosteres the secondary hydroxy group has to be protected, e.g. in the form of an oxazolidinone 24a.

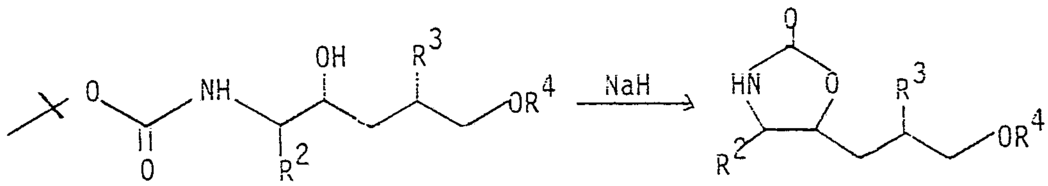


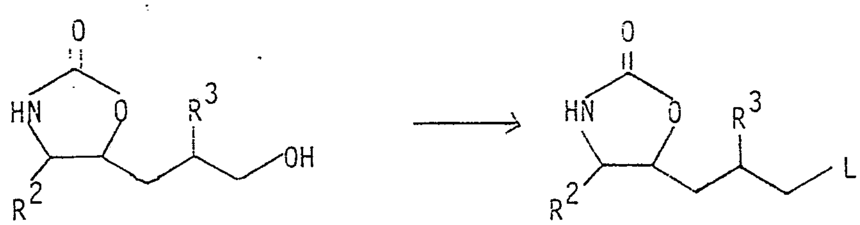

24 a

The oxazolidinone ring can then be cleaved to give the free amino alcohol 33.

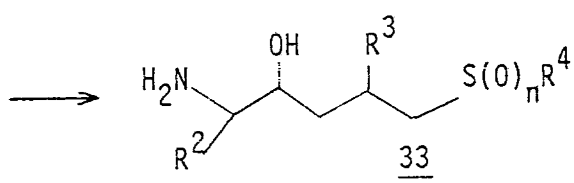

24a 33

II. Coupling of the isosteres to the amino acids.
This is done by use of standard peptide synthesis techniques such as coupling the isosteres with the hydroxybenzotriazole and hydroxysuccinimide esters of the appropriate N-protected amino acids in two separate steps, the protecting group on the amino acid C preferably being t-Boc and the one on amino acid B being X. In the cases where B is a dibenzylacetyl group standard amide forming reactions are applied. Alternatively, the isostere can be coupled with an activated acyl derivative of a X-B-C residue.

III. Separation of diastereomers.
In those cases where the reactions result in a mixture of diastereomers these are separated by standard chromatographic or recrystallization techniques.
The symbols used in the formulae above in paragraphs I-III refer to the following:

Z = O; $S(O)_n$
Mt = a metal
R = lower alkyl
$R^1$ = N-protecting group
$R^2$ = alkyl (1-6C); cyclohexylmethyl
$R^3$ = H; alkyl(1-6C)
$R^4$, = isopropyl
$R^9$ = amino or alkoxy residue
W = $H_2$; O
L = a leaving group,

D = 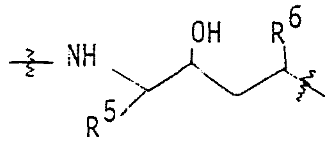

with or without any amino acid residues attached to the nitrogen.
n = O or 2

In a further aspect the invention is related to a compound of the formula

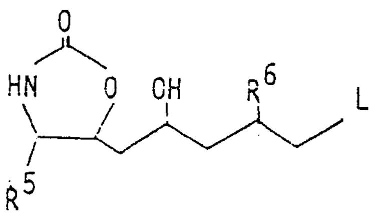

Xa

wherein L is hydroxy or a leaving group or an acid addition salt thereof, useful as an intermediate in the synthesis of a compound as defined in claim 1.

A further objective of the invention is a pharmaceutical composition containing the compounds of the invention. Thus they can be formulated for use in the reduction of blood pressure in compositions such as tablets, capsules or exilirs for oral or rectal administration or in sterile solutions or suspensions for parenteral or nasal administration. About O,OO1 to 5O mg of a compound is then compounded with a physiologically acceptable vehicle, carrier, excipient, binder, preservative, stabilizer, flavour etc. in a unit dosage form as called for by accepted pharmaceutical practice. The amount of active compound in these preparations is such that a suitable dosage in the range indicated is obtained. Variations and changes which are obvious to one skilled in the art of formulations are intended to be within the scope of the invention.

A further objective of the invention is the use of the compounds as drugs for treatment of hypertension, congestiveheart failure or other cardiovascular disorders. Thus for example by administration of a composition containing one of the compounds of the invention as active ingredient to a patient suffering from hypertension a reduction in blood pressure is obtained. The substance is preferably administered orally, but parenteral, rectal and nasal routes can also be used. The dosage is preferably O.OO1 to 5O mg of active ingredient and is preferably administered 1-3 times a day.

Best mode of carrying out the invention

The following examples illustrates the principles of the invention in more detail.

Example 1

Preparation of

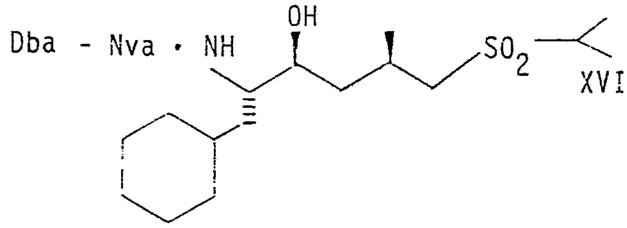

XVII

a) Preparation of

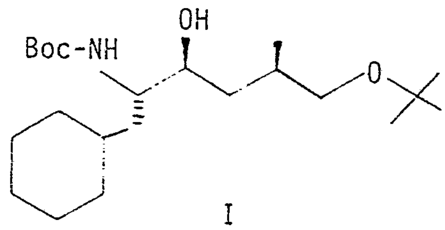

I

The intermediate II was prepared according to N. Cohen et al., J. Org. Chem. 41, 3505 (1976) and intermediate III was prepared according to J. Boger et al., J. Med. Chem. 28, 1779 (1985).

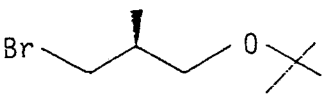

II

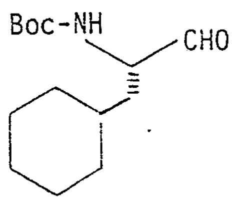

III

To a stirred mixture of 1.4 g (0.057 mol) of powdered magnesium in 14 ml of anhydrous THF were added a crystal of iodine and a few drops of a solution of 9.9 g (0.048 mol) of II in 20 ml of dry THF. The mixture was brought to reflux and after the reaction had started the remainder of the solution of II was added at such a rate that reflux was maintained. After the addition was complete, the reaction mixture was stirred at reflux for an additional 1 h, then cooled to 0-5°C whereupon a solution of 4.9 g (0.019 mol) of III in 35 ml of dry THF was added dropwise while keeping the temperature below 10°C. The reaction mixture was stirred for 3 h at room temperature and was then poured onto 30 ml of saturated ammonium chloride solution and 30 ml of water. 120 ml of ether was added and the mixture was stirred vigorously. The aqueous layer was ectracted with 2×40 ml of ether. The combined organic layers were dried over sodium sulfate and the ether was evaporated. The crude product was chromatographed on silica gel and eluted with petroleum ether/ethyl acetate 5:1. Yield: 3.4 g (46 %) of Ia and 0.65 g (9 %) of Ib.

b) Preparation of

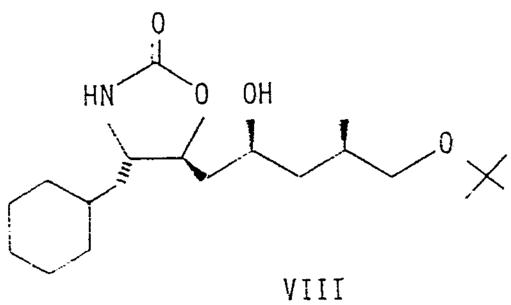

VIII

To a solution of 1.5 g (3.9 mmol) of I in 15 ml of dimethylformamide was added 0.24 g (10 mmol) of sodium hydride (55 % dispersion in mineral oil). The mixture was stirred for 5 h at room temperature and then poured onto 7 ml of saturated ammonium chloride solution and 7 ml of water. The mixture was extracted with 3×30 ml

of methylene chloride. The combined organic layers were dried over sodium sulfate and evaporated to give 1.1 g (91 %) of VIII which was used without further purification in the next step.

c) Preparation of

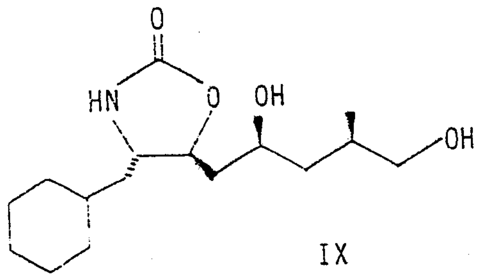

IX

A solution of 1.1 g of VIII in 6 ml of 4 M HCl and 6 ml of dioxan was heated on a 90°C water bath for 1 h. The reaction mixture was evaporated and then chromatographed on silica gel with ethyl acetate. Yield: 0.50 g (56 %) of IX. Alternatively, VIII can be treated with 2 eq. of trimethylsilyl iodide in methylene chloride for 30 min. The organic phase is then washed with $Na_2S_2O_3$(aq), water and dried ($MgSO_4$) giving the product.

d) Preparation of

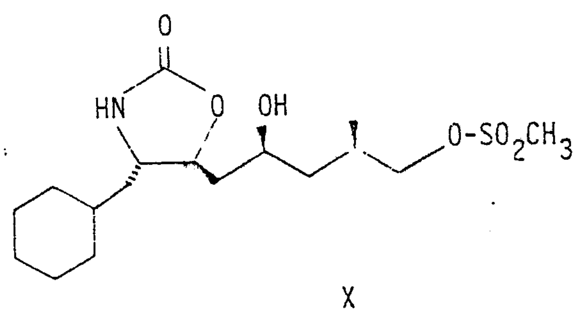

X

A solution of 0.50 g (1.9 mmol) of IX and 0.23 g (2.2 mmol) of triethylamine in 5 ml of methylene chloride was cooled to 0°C. Methanesulfonyl chloride, 0.24 g (2.1 mmol), was added. The reaction mixture was stirred for 2 h and the reaction was monitored by TLC. A 5 ml portion of petroleum ether (40-60°) was added and triethylammonium chloride was filtered off. The solution was evaporated to give 0.75 g (100 %) of X which was used directly in the next step.

e) Preparation of

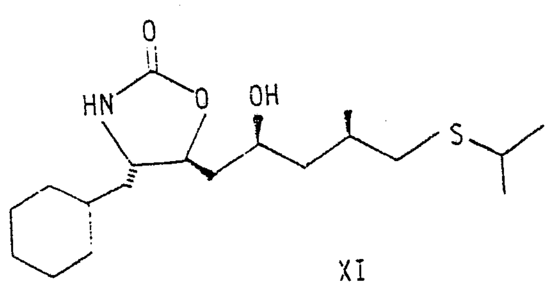

XI

To a solution of 0.75 g (1.9 mmol) of X in 5 ml of dry THF was added a suspension of sodium isopropyl thiolate - generated from 0.18 g (2.3 mmol) of isopropylthiol and 0.11 g (2.5 mmol) of sodium hydride (55 % dispersion in mineral oil) - in 5 ml of dry THF. The reaction mixture was stirred for 2 h. The reaction was monitored by TLC. Then 15 ml of methylene chloride and 5 ml of water were added and the mixture was vigorously stirred. The two layers were separated and the aqueous one was extracted with 5 ml of methylene chloride. The combined organic layers were dried over sodium sulfate and then evaporated to give 0.62 g (100 %) of XI which used directly in the next step.

f) Preparation of

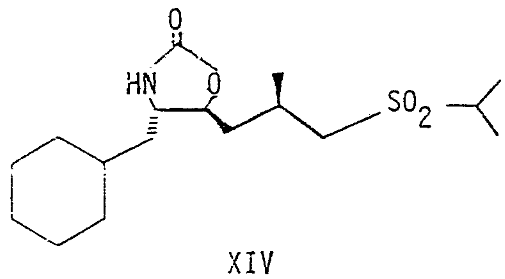

XIV

A solution of 0.50 g (1.6 mmol) of XI and 1.0 g of 55% meta-chloroperbenzoic acid (3.2 mmol) in 20 ml of methylene chloride was stirred for 3 h at room temperature. The mixture was then washed with 50 ml of 1M NaOH, then with water and finally dried over sodium sulfate and evaporated. The crude product was flash chromatographed on silica gel with petroleum ether-ethyl acetate 1/1.
Yield: 0.43 g (78%).

g) Preparation of

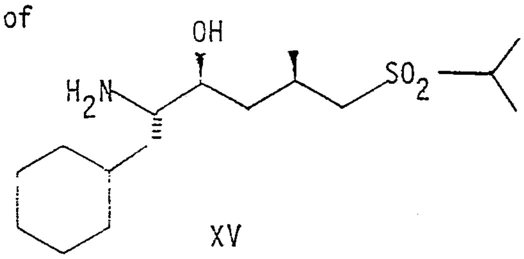

XV

A suspension of 0.43 g (1.2 mmol) of XIV and 0.28 g (5.0 mmol) of potassium hydroxide in 10 ml of ethanol was stirred and heated to 100°C overnight. The reaction mixture was poured into water and extracted with methylene chloride three times. The combined organic fractions were washed with water, dried over sodium sulfate and evaporated. The crude product started to crystallize and was used as such in the ensuing step.
Yield: 0.34 g (86 %).

h) Preparation of

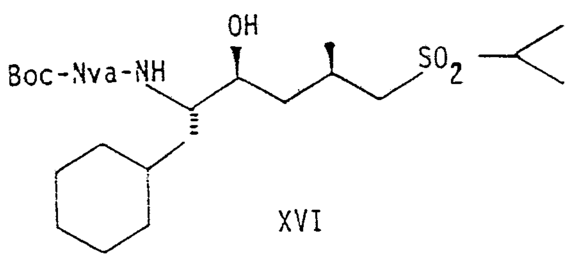

XVI

A mixture of 0.29 g (1.4 mmol) of Boc-Nva-OH and 0.37 g (2.7 mmol) of N-hydroxybenzotriazole in 6 ml of methylene chloride was cooled to 0°C. N-Cyclohexyl-N′-(2-morpholinoethyl)carbodiimide-metho-p-toluene-sulfonate, CME-CDI, 0.66 g (1.6 mmol), was added. The mixture was allowed to reach room temperature and was stirred for 3 h. The solvent was evaporated and a solution of 0.34 g (1.1 mmol) of XV in 5 ml of dimethylformamide was added and the pH adjusted to 8 by addition of N-methylmorpholine. The mixture was stirred at room temperature overnight. It was then poured into water and extracted three times with 50 ml portions of ethyl acetate. The combined organic phase was washed twice with 50 ml of 0.5M HCl, once with 50 ml of 1M NaOH and finally with water, dried over sodium sulfate and evaporated. The crude product was used as such in the next step. Yield: 0.41 g (75 %).

i) Preparation of XVII.

The hydroxybenzotriazole ester of dibenzylacetic acid was prepared from 0.21 g (0.87 mmol) of dibenzylacetic acid, 0.24 g (1.7 mmol) of N-hydroxybenzotriazole and 0.42 g (0.99 mmol) of CME-CDI in 10 ml of methylene chloride as described in the previous step.
Compound XVI, 0.41 g (0.79 mmol), was dissolved in 1.8 ml of methylene chloride and 0.6 ml of trifluoroacetic acid was added. After 1.5 h the mixture was evaporated, dissolved in 5 ml of dimethylformamide and added to the avaporated mixture above. The pH was adjusted to 8 by addition of N-methylmorpholine and the reaction was left stirring for 60 h. The same work-up procedure was used as in the previous step. The crude product was flash chromatographed on silica gel with petroleum ether-ethyl acetate 1/1. Yield: 0.33 g (65 %).

Example 2

Preparation of

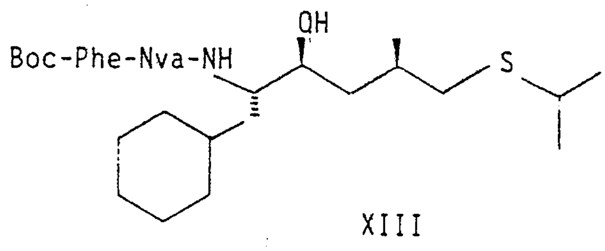

XIII

a) Preparation of

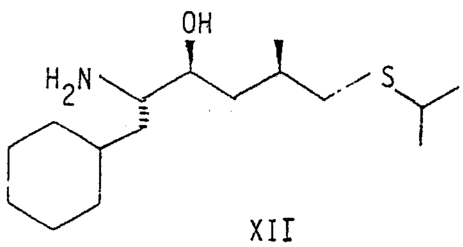

XII

A suspension of 0.62 g (1.9 mmol) of XI and 6.2 g barium hydroxide in 10 ml of water and 10 ml of dioxan was stirred and heated to 100°C for 4 h. The reaction mixture was cooled to room temperature and methylene chloride was added. The liquid layers were separated from the solid residue which was extracted several times with methylene chloride. The combined organic layers were dried over sodium sulfate, filtered and evaporated. The product was purified on silica, eluting with EtOAc:MeOH:HOAc:$H_2O$ 48:3:3:2.
Yield: 0.47 g (81 %) of XII.

b) Preparation of XIII.

The target compound was synthesized by standard peptide couplings with Boc-Nva-OH and Boc-Phe-OH. The yields in the reactions were 62 % and 35 % respectively.

Examples 3-6 (see tables 2 and 3)

The preparation of these compounds were accomplished in a similar way as described above in examples 1 and 2.

Example 7

Solution for injection

A solution is prepared from the following ingredients:

Renin inhibitor 1 g
Ethanol (99.5 %) 100 ml
Polyethylene glycol 400 400 ml
Water for inj. up to 1000 ml

The active constituent is dissolved in the ethanol/polyethylene glycol mixture. Water is added to final volume, whereafter the solution is filtered through a sterile 0,2 mikroM filter and aseptically filled into sterile ampoules (5 ml).

Example 8

Solution for injection

A solution is prepared from the following ingredients:

Renin inhibitor 1 g
Sodium chloride 9 g
Methyl p-hydroxybenzoate 0.5 g
Propyl p-hydroxybenzoate 0.2 g
Water for inj. up to 1000 ml

The active constituent, sodium chloride and preservatives are dissolved in the main part of the water, whereafter the volume is adjusted to 1000 ml. The solution is filtered through a sterile 0.2 mikroM filter and aseptically filled into sterile ampoules (5 ml).

Example 9

Solution for nasal administration

A solution is prepared from the following ingredients:

Renin inhibitor 10 g
Glycerol 200 g
Methyl p-hydroxybenzoate 1 g
Propyl p-hydroxybenzoate 0.2 g
Water for inj. up to 1000 ml

The active constituent and the preservatives were dissolved in glycerol and the main part of the water. The volume is then adjusted to 1000 ml and the solution is filled into sterile polyethylene containers.

Example 10

Tablets for oral administration

1000 tablets are prepared from the following ingredients:

Renin inhibitor 10 g
Lactose 100 g
Polyvinyl pyrrolidone 20 g
Avicel 20 g
Magnesium stearate 2 g

The active constituent and lactose are mixed withan aqueos solution of polyvinyl pyrrolidone. The mixture is dried and milled to form granules. The Avicel and then the magnesium stearate are then admixed. The mixture is then compressed in a tablet machine giving 1000 tablets, each containing 10 mg of active constituent.

Example 11

Gelatine capsules for oral administration

Gelatin capsules are filled with a mixture of the following ingredients:

Renin inhibitor 10 mg
Magnesium stearate 2 mg
Lactose 188 mg

Biological data

The potencies of the compounds of the invention as renin inhibitors were determined in vitro using a human renin/angiotensinogen reaction. The assay is based on the method of Ikeda et al (J. Clin. Endocrinal Metab. 54, 423 (1982)), and relies on a radioimmunometric determination of the amount of angiotensin I released from angiotensinogen by renin in a human plasma pool.

The compounds of the invention were dissolved in 0,1 M acetic acid (10 microliter) and then added to human plasma (200 microliter) containing EDTA and 0,6 M citrate buffer (20 microliter). After incubation for 60 minutes at 37°C, $^{125}$I-labelled angiotensin I containing 0,5 mg/ml pepstatine A was added. Antibody coated spheres were added and the resulting mixture incubated for 3 hours at room temperature. 2 ml of distilled water was then added, whereafter the liquid was removed with an aspirator. The radioactivity of the spheres was then determined using a gamma scintillation technique.

The potencies of the compounds thus obtained (see table 4) are given as means of at least four experiments and are expressed as $pIC_{50}$, i.e. the negative logarithm of the molar concentration required to cause 50 % inhibition.

Table 4

| Example no | $pIC_{50}$ |
|---|---|
| 1 | 8,3 |
| 2 | 8,0 |
| 3 | 9,3 |
| 4 | 6,7 |
| 5 | 8,2 |
| 6 | 7,4 |

Table 1

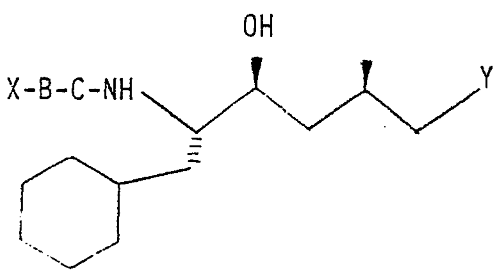

| X | B | C | Y |
|---|---|---|---|
| Boc | Phe | Nva | $SCH(CH_3)_2$ |
| Boc | Phe | Nva | $SO_2CH(CH_3)_2$ |
| H | Dba | Nva | $SO_2CH(CH_3)_2$ |
| Boc | Tyr(Me) | Nva | $SO_2CH(CH_3)_2$ |

Table 2. Summary of working examples

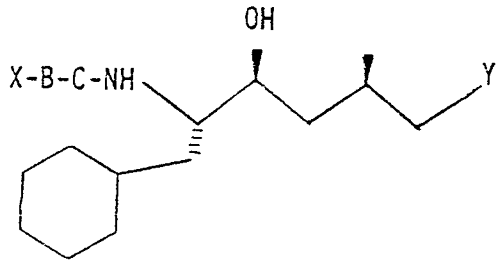

| Ex | X | B | C | Y | Method of preparing the isostere | Identifying data |
|---|---|---|---|---|---|---|
| 1 | H | Dba | Nva | $SO_2$ 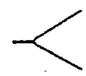 | A | $^1H$ NMR |
| 2 | Boc | Phe | Nva | S 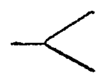 | A | $^1H$ NMR |
| 3 | Boc | Phe | Nva | $SO_2$ 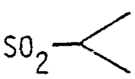 | A | $^1H$ NMR |
| 4 | Boc | Tyr(Me) | Nva | S 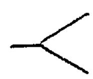 | A | $^1H$ NMR |
| 5 | Boc | Tyr(Me) | Nva | $SO_2$ 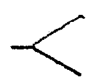 | A | $^1H$ NMR |
| 6 | Boc | Phe | Me-Nva | $SO_2$ 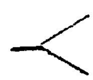 | A | $^1H$ NMR |

Identifying data for compounds of the invention.

| Ex | $^{1}$HNMR, 500 MHz: ppm ($CDCl_3$) |
|---|---|
| 1. | 0.6-2.0 (m,31H): thereof 0.70 (t,3H), 1.14 (d,3H), 1.14 (d,3H), 1.38 (d,3H), 1.40 (d,3H). 2.20 (m,1H); 2.3-3.12 (m,8H); 3.37 (m,1H); 3.59 (M,1H); 3.88 (d,1H); 4.20 (m,1H); 5.60 (d,1H); 5.87 (d,1H); 7.1-7.4 (m,10H). |
| 2. | 0.90(t,3H); 1.05(d,3H); 1.10-1.95(m,20H); 1.25(dd,6H); 1.40(s,9H); 2.40(dd,1H); 2.60(dd,1H); 2.90(m,1H); 3.00(dd,1H); 3.10(dd,1H); 3.65(m,1H); 3.90(m,1H); 4.30(m,2H); 4.90(d,1H); 6.35(bs,2H); 7.15-7.35(m,5H) |
| 3. | 0.7-1.9 (m,40H); thereof 0.91(t,3H), 1.21 (d,3H), 1.39 (d,3H), 1.41 (d,3H), 1.42 (s,9H); 2.41 (m,1H); 2.78 (dd,1H); 3.00 (dd,1H); 3.14 (m,3H); 3.63 (m,1H); 3.90 (m,1H); 4.27 (m,2H); 4.95 (bd,1H); 6.39 (m,2H); 7.1-7.4 (m,5H). |
| 4. | 0.8-2.0 (m,41H); thereof 0.93 (t,3H), 1.02 (d,3H), 1.23 (d,3H), 1.28 (d,3H), 1.45 (s,9H); 2.40 (dd,1H); 2.6-2.75 (m,2H); 2.8-3.15 (m,3H); 3.65 (m,1H); 3.81 (s,3H); 3.93 (m,1H); 4.11 (m,1H); 4.22 (m,1H); 4.32 (m,1H); 4.91 (bd,1H); 6.3-6.5 (m,2H); 6.81 (d,2H); 7.10 (d,2H) |
| 5 | 0.75-1.9 (m,40H); thereof 0.92 (t,3H), 1.21 (d,3H), 1.38 (d,3H), 1.41 (d,3H); 1.43 (s,9H); 2.44 (m,1H); 2.78 (dd,1H); 2.96 (dd,1H); 3.0-3.25 (m,4H); 3.62 (m,1H); 3.80 (s,3H); 3.93 (m,1H); 4.21 (m,1H); 4.26 (m,1H); 4.89 (m,1H); 6.30 (m,1H); 6.40 (m,1H); 6.87 (d,2H); 7.12 (d,2H) |

This compound exists as two conformers

0.7-2.0 (m,40H); thereof 0.87 (t,3H); 1.14 (d,3H), 1.35 (d,3H); 1.38 (s,9H); 1.40 (d,3H); 2.3-2.5 (m,1H); 2.6-3.2 (m,5H); 2.68 (s,3H, major conformer); 2.81 (s,3H, minor conformer); 3.59 (m,1H); 3.75-3.95 (m,1H); 4.00 (m,1H); 4.43 (m,1H, major conformer); 4.48 (m,1H, minor conformer); 4.57 (m,1H, major conformer); 4.90 (m,1H, minor conformer); 5.26 (d,1H); 7.1-7.4 (m,5H)

## Claims

1. Compounds of the general formula

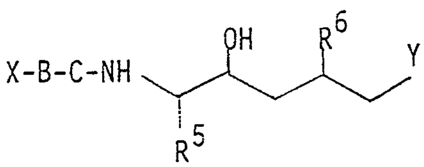

where
X is H or an N-protecting group
$R^1-(CH_2)_n-O-C(O)-$
where n is O-4; $R^1$ is a straight or branched alkyl group containing 1-6 carbon atoms
and
B is phenylalaninyl; O-methyltyrosinyl; homophenylalaninyl; cyclohexylalaninyl; dibenzylacetyl
and
C is norvalinyl; valinyl; norleucinyl; leucinyl; histidinyl; either as such or N -alkylated
and
$R^5$ is a straight or branched alkyl group containing 1-6 carbon atoms or a cyclohexylmethyl group
$R^6$ is H or a straight or branched alkyl group containing 1-6 carbon atoms
and
Y is selected from
a) $-SCH(CH_3)_2$
b) $-S(O)_2CH(CH_3)_2$
either as such or in the form of physiologically acceptable salts and including optical isomers.

2. Compounds according to claim 1 where X is selected from Boc and H, either as such or in the form of physiologically acceptable salts and including optical isomers.

3. Compounds according to claim 1 or 2 where B is selected from Phe, Tyr(Me), Cha, hPhe and Dba, either as such or in the form of physiologically acceptable salts and including optical isomers.

4. Compounds according to one or more of the preceding claims where C is selected from His, Nle, Nva, Me-Nva, Me-Nle and Me-His, either as such or in the form of physiologically acceptable salts and including optical isomers.

5. Compounds according to one or more of the preceding claims where $R^5$ is a cyclohexylmethyl group, either as such or in the form of physiologically acceptable salts and including optical isomers.

6. Compounds according to one or more of the preceding claims where $R^6$ is methyl, either as such or in the form of physiologically acceptable salts and including optical isomers.

7. Compounds according to one or more of the preceding claims where Y is $SCH(CH_3)_2$, either as such or in the form of physiologically acceptable salts and including optical isomers.

8. Compounds according to one or more of claims 1-7 where Y is $S(O)_2CH(CH_3)_2$, either as such or in the form of physiologically acceptable salts and including optical isomers.

9. A compound of the formula

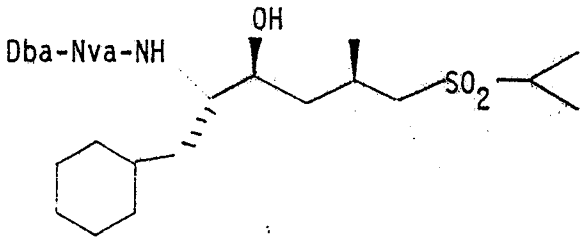

either as such or in the form of a physiologically acceptable salt and including optical isomers.

10. A compound of the formula

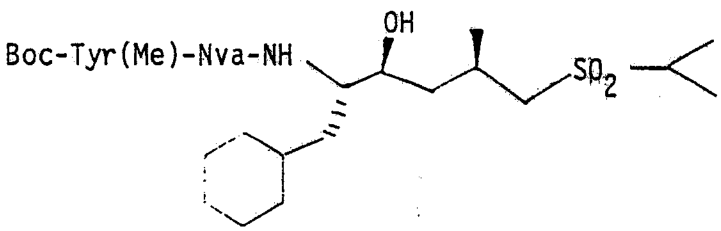

either as such or in the form of a physiologically acceptable salt and including optical isomers.

11. A compound of the formula

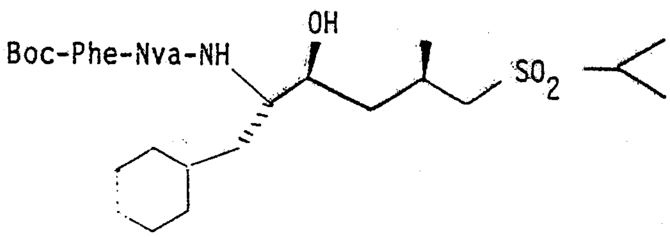

either as such or in the form of a physiologically acceptable salt and including optical isomers.

12. A compound of the formula

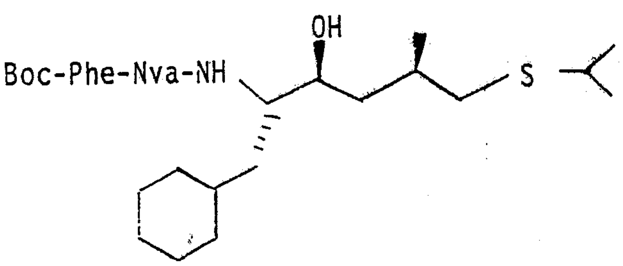

either as such or in the form of a physiologically acceptable salt and including optical isomers.

13. A process for preparation of compounds according to any of claims 1-12, wherein an isostere of the formula

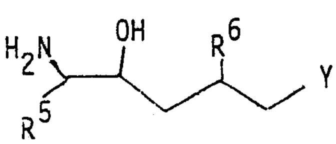

wherein $R^5$, $R^6$ and Y are as defined in any of claims 1-12, is coupled to appropriate amino acids by standard peptide synthesis techniques, and in those cases where the reactions result in a mixture of diastereomers these are separated by standard chromatographic or recrystallization techniques, and if

desired, forming a physiologically acceptable and/or isolating an optical isomer.

A process according to claim 13 wherein the starting material identified therein is prepared from a compound of the formula

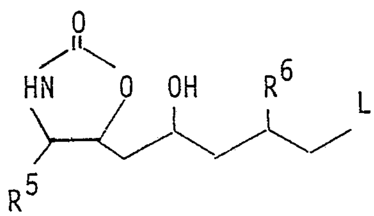

Xa

wherein L is a leaving group, by introducing sulfur to replace L with an isopropylthio group, and where required oxidizing the same to a sulfone, and by cleaving the oxazolidinore ring.

15. A compound of the formula

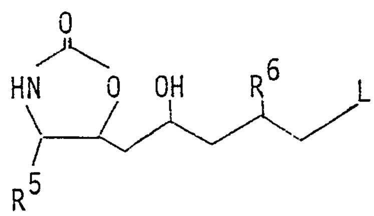

Xa

wherein L is hydroxy or a leaving group, or an acid addition salt thereof.

16. Use of a compound as defined in claim 15 as a starting material or an intermediate in the synthesis of renininhibiting peptide analogues.

17. A compound according to any of the claims 1-12 for use as a renin inhibitor.

18. A compound according to any of claims 1-12 for use as a drug for treatment of hypertension, congestive heart failure and other cardiovascular disorders.

19. A pharmaceutical preparation for treatment of hypertension, congestive heart failure and other cardiovascular disorders comprising a therapeutically effective amount of any of the compounds outlined in claims 1-12, and furthermore comprising a pharmaceutical carrier.

20. Use of a compound according to any of claims 1-12 as an active ingredient for manufacture of a pharmaceutical preparation for obtaining inhibition of renin in a human or animal organism.

21. Use of a compound according to any of the claims 1-12 as an active ingredient for manufacture of a pharmaceutical preparation for treatment of hypertension, congestive heart failure and other cardiovascular disorders.

22. A method of treatment of hypertension, congestive heart failure or other cardiovascular disorders, comprising administrating to a host in need of such treatment a therapeutically effective amount of a compound claimed in any of claims 1-12.

23. A compound, a process, a pharmaceutical preparation, use and a method as claimed in any of claims 1-22 and substantially as described.